# EUROPEAN PATENT APPLICATION

(11) **EP 2 218 794 A1**
(43) Date of publication of application: **18.08.2010**
(21) Application number: 10153547.4
(22) Date of filing: 12.02.2010
(51) Int. Cl.: C12Q 1/68

(54) **Detection of instability in regions of genomic DNA containing simple tandem repeats**

(30) Priority: 13.02.2009 CH 2332009
(71) Applicant: Alphagenics International SA, 1052 Le Mont (CH)
(72) Inventor: Morandi, Luca, 40126 Bologna (IT)
(74) Representative: Grosfillier, Philippe

(57) **Abstract**

A method of analyzing micro-satellite loci, comprising steps of: a) providing primers for co-amplifying a set of at least 13 microsatellite loci of human genomic DNA, comprising at least six mononucleotide repeat loci selected from the group consisting of BAT-25, BAT-26, BAT-40, MybT22, TGF-Beta R2 and MTIXT20 and at least four dinucleotide repeat loci selected from the group consisting of D18S58, D2S123, D5S346, D17S250 and at least three tetranucleotide repeat loci selected from the group consisting of D18S51, D7S820 and CSF1PO; b) co-amplifying the set of at least six microsatellite loci from at least one sample of genomic DNA in a multiplex amplification reaction, using at least one oligonucleotide primer for each locus which is fluorescently labeled, thereby producing amplified DNA fragments; and c) determining the size of the amplified DNA fragments

## Description

### FIELD OF INVENTION

This invention relates to the detection of instability in regions of genomic DNA containing simple tandem repeats, such as microsatellite loci. The invention particularly relates to multiplex analysis for the presence or absence of instability in a set of microsatellite loci in genomic DNA from cells, tissue, or bodily fluids originating from a tumor. The invention also relates to the use of microsatellite instability (MSI) analysis in the detection and diagnosis of cancer, predisposition for cancer, prognosis related to a determined pattern of MSI or loss of heterozygosity. The invention also provide information to predict a benefit from 5FU-based chemotherapy, and thus MSI might be a useful molecular predictive marker for response to this type of adjuvant therapy. The invention particularly relates to the identification of a new MSI mononuclotide marker with T-20 repeats located in the 3'-UTR of the MT1X gene, to use in combination with other 13 known microsatellite loci.

### BACKGROUND OF THE INVENTION

Colorectal carcinoma (CRC) is the second leading cause of cancer-related deaths in the Western world. There are -110,000 new cases and 50,000 deaths due to the disease per year in the U.S. A small fraction of patients (∼5%) have a hereditary colorectal cancer syndrome [Kinzler KW, Vogelstein B. Cell. 1996; 87:159-70]. One of these syndromes, hereditary non-polyposis colorectal cancer (HNPCC), accounts for 1% to 2% of all colorectal carcinomas [Salovaara R, Loukola A, Kristo P, et al. J Clin Oncol. 2000; 92:2193-200]. In most hNPCC colorectal tumors, MSI has been shown to result from defects in DNA mismatch repair. Mutations in the hMLH1 or hMSH2 genes are the most common defects in these families; in equal proportions, these make up about 94% of the germ line mutations detected. About 10% to 15% of sporadic colorectal cancers also exhibit MSI, and loss of expression of one or more of the MMR proteins has been found in these tumors [Chaves P, Cruz C, Lage P, et al. J Pathol. 2000; 191: 355- 60]. A microsatellite locus is a region of genomic DNA with simple tandem repeats that are repetitive units of one to five base pairs in length. Hundreds of thousands of such microsatellite loci are dispersed throughout the human genome. Microsatellite loci are classified based on the length of the smallest repetitive unit. For example, loci with repetitive units of 1 to 5 base pairs in length are termed "mononucleotide", "dinucleotide", "trinucleotide", "tetranucleotide", and "pentanucleotide" repeat loci, respectively. Most sporadic MSI-positive tumors lack expression of hMLH1, as the result of promoter methylation [Herman JG, Umar A, Polyak K, et al. Proc Natl Acad Sci U S A. 1998; 95:6870- 5]. MSI is an useful tool in identifying patients with HNPCC and sporadic CRC with defective DNA mismatch repair (MMR) genes. Moreover, the assessment of MSI status may be useful in establishing the oncological outcome of CRC patients and also in predicting tumor response to chemotherapy. In 1997, a reference panel of 5 markers was suggested for MSI testing by the National Cancer Institute aiming to standardize the test [Boland CR, Thibodeau SN, Hamilton SR et al. Cancer Res. 1998 Nov 15;58(22):5248-57], however this panel has limitations resulting from the inclusion of dinucleotide markers, which are less sensitive and specific for detection of tumors with MMR deficiencies compared to other markers that are currently available. Based on the number of microsatellites found to be unstable, tumors are classified as stable, with low instability (MSI-L) and with high instability (MSI-H). Dinucleotide repeats in the Bethesda panel (D2S123, D17S250 and D5S546) generally show instability in only 60%-80% of MSI-H tumors [Sutter C, Gebert J, Bischoff P et al.. Mol Cell Probes. 1999; 13:157-65]. Two dinucleotide markers (using the Bethesda reference panel) are observed to be unstable in the absence of BAT-26 deletions [Loukola A, Eklin K, Laiho P et al. Cancer Res. 2001; 61:4545-9]. Hoang JM et Al. [Cancer Res 1997;57:300-3] used only the analysis of mononucleotide repeats BAT-25 and BAT-26 to establish MSI status without reference to the germline DNA, because these markers are quasi-monomorphic, however stability in BAT-26 locus is strongly evocative for the presence of wide intragenic deletion in the *MSH2* gene and polymorphic BAT-25 and BAT-26 alleles have been identified in 18.4% and 12.6%, respectively, of Afro-Americans and in a small percentage of Caucasian individuals [Sood AK, Holmes R, Hendrix MJ et al.Cancer Res. 2001; 61:4371-4]. Additionally a panel of five quasimonomorphic mononucleotide repeats that dispenses with the need to analyse corresponding germline DNA was proposed by Suraweera *et al* [Suraweera N, Duval A, Reperant M, et al. Gastroenterology 2002; 12:1804-11] for the detection of high-frequency microsatellite instability (MSI) in colorectal cancer in a Caucasian study population in which no matching normal DNA would be available without microdissection. The present invention was developed to evaluate accurately MSI status following the Bethesda guidelines [Umar A, Boland CR, Terdiman JP et al.J Natl Cancer Inst. 2004; 18:261-8]. There has been considerable controversy about how to precisely define and accurately measure MSI (Boland, 1998 CancerResearch 58:5248-5257). Reports on the frequency of MSI in various tumors ranges considerably. For example, different studies have reported ranges of 3% to 95% MSI for the frequency of MSI in bladder cancer (Gonzalez-Zulueta et al., 1993 Cancer Research 53:28-30; Mao et al., 1996 PNAS 91:9871-9875). One problem with defining MSI is that it is both tumor specific and locus dependent (Boland et al. 1998, CancerResearch 58:5248-5257). Thus, the frequency of MSI observed with a particular tumor type in a single study will depend on the number of tumors analyzed, the number of loci investigated, how many loci need to be altered to score a tumor as having MSI and which particular loci were included in the analysis. To help resolve these problems, the National Cancer Institute sponsored a workshop on MSI to review and unify the field. As a result of the workshop a panel of five microsatellite was recommended as a reference panel for future research in the field. This panel included two mononucleotide loci BAT-25, BAT-26, and three dinucleotide loci D5S346, D2S123, D17S250. One particular problem in MSI analysis of tumor samples occurs when one of the normal alleles for a given marker is missing due to LOH, and no other novel fragments are present for that marker. One cannot easily discern whether this represents true LOH or MSI in which the shifted allele has co-migrated with the remaining wild-type allele. In cases like this, the recommendation from the NCI workshop on MSI was not to call it as MSI. One way to minimize this type of problem would be to use loci that displayed low frequency of LOH in colon tumors. Clinical diagnostic assays used for determining treatment and prognosis of disease require that the tests be highly accurate (low false negatives) and specific (low false positive rate). Many informative microsatellite loci have been identified and recommended for MSI testing (Boland et al. 1998, CancerResearch 58:5248-5257). However, even the most informative microsatellite loci are not 100% sensitive and 100% specific. To compensate for the lack of sensitivity using individual markers, multiple markers can be used to increase the power of detection. The increased effort required to analyze multiple markers can be offset by multiplexing. Multiplexing allows simultaneous amplification and analysis of a set of loci in a single tube and can often reduce the total amount of DNA required for complete analysis. To increase the specificity of an MSI assay for any given type of cancer it has been recommended that the panel of five highly informative microsatellite loci identified at the National Institute Workshop (Boland et al. CancerResearch 58:5248-5257) be modified to add other loci of equal utility. Increased information yielded from amplifying and analyzing greater numbers of loci results in increased confidence and accuracy in interpreting test results.
CSF1PO, D18S51 and D7S820 are tetranucleotide microsatellite markers commonly used for forensic purposes. They are usually stable even in MSI-H tumors and they are useful to detect contaminating DNA if present helping confirm that tumor and matching normal samples are from the same individual. Chromosome 18q allelic loss has been reported to have prognostic significance in stage II colorectal carcinoma. D18S51 and D18S58 map at the long arm of chromosome 18 close to each other and both markers are useful to acquire additional informations chromosome 18q allelic loss.

The materials and methods of the present invention are designed for use in multiplex analysis of a set of 13 microsatellite loci of human genomic DNA from various sources, including various types of tissue, cells, and bodily fluids. The present invention represents a significant improvement over existing technology, bringing increased power of discrimination, precision, and throughput to the analysis of MSI loci, and prognosis related to a determined pattern of MSI or loss of heterozygosity. The invention also provide information to predict a benefit from 5FU-based chemotherapy, and thus MSI might be a useful molecular predictive marker for response to this type of adjuvant therapy. The invention particularly relates to the identification of a new MSI mononuclotide marker with T-20 repeats located in the 3'-UTR of the MT1X gene.

### GENERAL DESCRIPTION OF THE INVENTION

The present invention provides methods and kits for amplifying and analyzing a sets of microsatellite loci following National Institute Workshop (Boland et al. CancerResearch 58:5248-5257) adding to classical BAT26, BAT25, D2S123, D5S346 and D17S250, the following markers: BAT-40, MybT22, TGF-Beta R2, MT1XT20, D18S58, D18S51, D7S820 and CSF1PO. The present invention also provides methods and kits for detecting cancer in an individual by co-amplifying multiple microsatellite loci of human genomic DNA originating from tumor tissue or cancer cells.

The present invention provides a method of analyzing microsatellite loci, comprising: (a) providing primers for co-amplifying a set of six microsatellite loci in one reaction tube and a set of seven microsatellite loci in another reaction tube , from a first sample originating from normal non-cancerous biological material from an individual and a second sample originating from cancerous material from the same individual. The at least two samples of human genomic DNA are co-amplified in separate multiplex amplification reactions, using primers specified below. The size of the resulting amplified DNA fragments from the two multiplex reactions from normal and the two from cancerous DNA are compared to one another to detect instability and loss of heterozygosity in any of the tested loci.

The present invention relates in particular to a kit for performing a method for assessment of the MSI status of medically relevant conditions associated with MSI comprising one or more binding agents specifically recognizing a MT1X nucleic acid.

Preferably at least one binding agent is a nucleic acid probe or primer.

The probes or primers may be selected from SEQ ID NO: 1-26 (see below).

The invention also relates to a kit for performing a method for assessment of the MSI status of medically relevant conditions associated with MSI as defined above and comprising a pair of primers that allow for specific amplification of a nucleic acid comprising the T-20 repeat located in the 3'-UTR of the MI1X nucleic acid.

With this kit the nucleic acid may range in length from 50 nt to up to 500 nt.

The kit may be provided as in- vitro diagnostic device.

The kit may comprise agents for the detection of one or more further microsatellite markers.

Advantageously one or more further microsatellite markers are selected from a group comprising the mononucleotide markers BAT-25, BAT-26, BAT-40, MybT22, TGF-Beta R2 and MT1XT20 and the dinucleotide repeat loci selected from the group consisting of D18S58, D2S123, D5S346, D17S250 and the tetranucleotide repeat loci selected from the group consisting of D18S51, D7S820 and CSF1PO.

The kit may comprise agents for detection of polymorphic typing markers as control reagents.

The invention also concerns the use of primers or probes complementary or reverse complementary to MT1X nucleic acids or fragments for development and/or manufacture of a kit for performing a method for assessment of the MSI status of medically relevant conditions associated with MSI

The invention also encompasses a system for assessment of the MSI status of medically relevant conditions based on the detection of mutations in the T-20 repeat of the 3'-UTR of the MT1X gene comprising one or more components including but not limited to at least one component for detection and/or evaluation of signal levels and/or intensities, wherein the signals are indicative for the presence or absence of mutations in the T-20 repeat of the 3'-UTR of the MT1X gene; and at least one component for correlation of the detected and/or evaluated signal levels and/or intensities to clinical data, wherein the clinical data pertain to expectation for success and/or effectiveness of therapeutic measures applied to the medically relevant condition under investigation.

The system maybe computer aided.

The invention also covers a computer implemented method for building a strategy for therapy of medically relevant conditions associated with MSI comprising i) comparing data representing the presence or absence of mutations in the T-20 repeat located in the 3'-UTR of the MT1X gene to clinical data comprising information on the presence or absence of mutations in said T-20 repeat and information about the patients disease course and outcome by a comparing means; ii) building up subgroups of data representing individuals according to the presence or absence of mutations and giving subgroups to an assessment means, and iii) assessing the comparison based on the subgroups of data by the assessment means and giving the assessment from the assessment means to a therapy tailoring means, the therapy tailoring means tailoring an adequate therapy for the individuals according the assessment.

Objet of the invention is also a pair of nucleic acid primers complementary or reverse complementary to the MT1X gene that are designed to allow for amplification of a nucleic acid 50 to 500 nt in length comprising the T-20 repeat located in the 3'-UTR of the MT1X gene for use in a method of assessment of the MSI status of medically relevant conditions associated with MSI as defined previously.

The system according to the invention may provide information about loss of heterozygosity for D18S58 and D18S51 for correlation of the detected and/or evaluated signal levels and/or intensities to clinical data, wherein the clinical data pertain to prognosis in patients with stage II cancer, patients with stage III cancer, or both groups,

The system may also provide information about MSI status and TGF-BetaR2 for correlation of the detected and/or evaluated signal levels and/or intensities to clinical data, wherein the clinical data pertain to prognosis in patients with stage II cancer, patients with stage III cancer, or both groups.

**Table of markers used:**

| Marker | Multiplex | Capillary electrophoresis group | Chromosome map | Repeats | Length (bp) | Primer Dyes |
|---|---|---|---|---|---|---|
| BAT26 | 1 | 1 | 2p21-22 | A₍₂₆₎ | 122-128 | D4/HEX |
| D17S250 | 1 | 1 | 17q12 | CA₍ₙ₎ | 144-158 | D4/FAM |
| TGF-BetaR | 1 | 1 | 3p22 | A(n) | 172-176 | D3/HEX |
| D2S123 | 1 | 1 | 2p15 | CA₍ₙ₎ | 206-220 | D4/FAM |
| D5S346 | 1 | 1 | 5q22-23 | CA₍ₙ₎ | 250-265 | D4/HEX |
| CSF1PO | 1 | 1 | 5q33.3-34 | TGAT₍ₙ₎ | 299-323 | D4/FAM |
| MybT22 | 2 | 2 | 6q22-q23 | T₍₂₂₎ | 90-100 | D3/HEX |
| BAT25 | 2 | 2 | 4q12 | T₍₂₅₎ | 108-115 | D4/FAM |
| D18S58 | 2 | 2 | 18q23 | CA₍ₙ₎ | 159-169 | D3/HEX |
| Mt1xT20 | 2 | 2 | 16q13 | T₍₂₀₎ | 180-190 | D4/FAM |
| D7S820 | 2 | 2 | 7p21.1 | TAGA₍ₙ₎ | 198-222 | D3/HEX |
| BAT40 | 2 | 2 | 1p12-13.3 | A₍₄₀₎ | 240-255 | D4/FAM |
| D18S51 | 2 | 2 | 18q21.33 | GAAA₍ₙ₎ | 274-318 | D4/HEX |

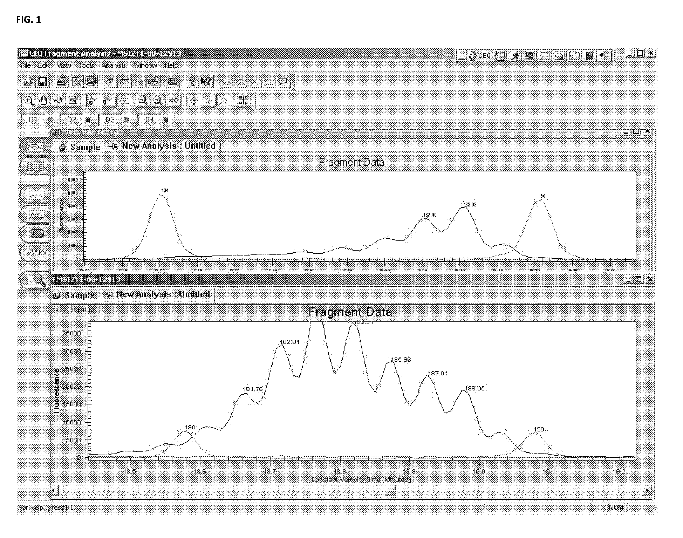

Another embodiment of the present invention is a method of analyzing only MT1XT20 repeat locus of human genomic DNA and comprises the steps of: (a) providing at least one primer of the at least one mononucleotide repeat locus; (b) amplifying the MTIXT20 locus from a sample of genomic DNA originating from a normal and cancerous biological material from an individual human subject, using the at least one primer, thereby producing an amplified DNA fragment; and (c) determining the size of the amplified DNA fragments. The amplified DNA fragments are preferably analyzed to detect microsatellite instability by comparing the size of the amplified DNA fragments of normal non-cancerous against the amplified DNA fragments obtained from cancerous DNA. The method and kit can also be used to compare the results of multiplex amplification of DNA from normal tissue of an individual to the results of multiplex amplification of DNA from other biological material from the same individual. Use of this particular embodiment of the method of the present invention to detect MSI in tumor cells by comparison to normal cells is illustrated in FIG. 1.

Fig.1 illustrates microsatellite instability detected comparing normal tissue (upper fragment data) respect to tumor tissue (lower fragment data) using MT1XT20 marker:

### Definitions

The following definitions are intended to assist in providing a clear and consistent understanding of the scope and detail of the following terms, as used to describe and define the present invention:
- "Allele", as used herein, refers to one of several alternative forms of a gene or DNA sequence at a specific chromosomal location (locus). At each autosomal locus an individual possesses two alleles, one inherited from the father and one from the mother.
- "Amplify", as used herein, refers to a process whereby multiple copies are made of one particular locus of a nucleic acid, such as genomic DNA. Amplification can be accomplished using any one of a number of known means, including but not limited to the polymerase chain reaction (PCR) (Saiki, R. K., et al., 1985 Science 230: 1350-1354), transcription based amplification (Kwoh, D. Y., and Kwoh, T. J., American Biotechnology Laboratory, October, 1990) and strand displacement amplification (SDA) (Walker, G. T., et al., 1992 Proc. Natl. Acad. Sci., U.S.A. 89: 392-396).
- "Co-amplify", as used herein, refers to a process whereby multiple copies are made of two or more loci in the same container, in a single amplification reaction. "DNA polymorphism", as used herein, refers to the existence of two or more alleles for a given locus in the population. "Locus" or "genetic locus", as used herein, refers to a unique chromosomal location defining the position of an individual gene or DNA sequence.
- "Locus-specific primer", as used herein, refers to a primer that specifically hybridizes with a portion of the stated locus or its complementary strand, at least for one allele of the locus, and does not hybridize efficiently with other DNA sequences under the conditions used in the amplification method.
- "Loss of Heterozygosity" (LOH), as used herein, refers to the loss of alleles on one chromosome detected by assaying for markers for which an individual is constitutionally heterozygous. Specifically, LOH can be observed upon amplification of two different samples of genomic DNA from a particular subject, one sample originating from normal biological material and the other originating from a tumor or pre-cancerous tissues. The tumor exhibits LOH if DNA from the normal biological material produces amplified alleles of two different lengths and the tumor samples produces only one of the two lengths of amplified alleles at the same locus. "Microsatellite Locus", as used herein, refers to a region of genomic DNA that contains short, repetitive sequence elements of one (1) to seven (7), more preferably one (1) to five (5), most preferably one (1) to four (4) base pairs in length. Each sequence repeated at least once within a microsatellite locus is referred to herein as a "repeat unit." Each microsatellite locus preferably includes at least seven repeat units, more preferably at least ten repeat units, and most preferably at least twenty repeat units.
- "Microsatellite instability" (MSI), as used herein, refers to a form of genetic instability in which alleles of genomic DNA obtained from certain tissue, cells, or bodily fluids of a given subject change in length at a microsatellite locus. Specifically, MSI can be observed upon amplification of two different samples of genomic DNA from a particular subject, such as DNA from healthy and cancerous tissue, wherein the normal sample produces amplified alleles of one or two different lengths and the tumor sample produces amplified alleles wherein at least one of the alleles is of a different length from the amplified alleles of the normal sample of DNA at that locus. MSI generally appears as a result of the insertion or deletion of at least one repeat unit at a microsatellite locus.
- "MSI-H" (High), as used herein, is a term used to classify tumors as having a high frequency of MSI. As previously described by Boland et Al. (Boland, 1998 Cancer Research 58: 5248-5257), when more than five microsatellite loci are analyzed, a tumor is classified as MSI-H when at least 30% of the microsatellite loci of genomic DNA originating from the tumor are found to be unstable.
- "MSI-L" (Low), as used herein, is a term used to classify tumors as having a low frequency of MSI. When five microsatellite loci are analyzed, such as the five microsatellite loci of selected by a workshop on HNPCC at the National Cancer Institute in 1998 for use in the detection of HNPCC, a tumor is classified as MSI-L when only one of the loci shows instability. When more than five microsatellite loci are analyzed, a tumor is classified as MSI-L when less than 30% of the microsatellite loci of genomic DNA originating from the tumor is are found to be unstable. MSI-L tumors are thought to represent a distinct mutator phenotype with potentially different molecular etiology than MSI-H tumors (Thibodeau, 1998; Wu et al., 1999, Am J Hum Genetics 65:1291-1298). To accurately distinguish MSI-H and MSI-L phenotypes it has been recommended that more than five microsatellite markers be analyzed (Boland, 1998, supra; Frazer et al., 1999 Oncology Research 6:497-505).
- "MSS", as used herein, refers to tumors which are microsatellite stable, when no microsatellite loci exhibit instability. The distinction between MSI-L and MSS can also only be accomplished when a significantly greater number of markers than five are utilized. The National Cancer Institute recommended use of an additional 19 mono- and dinucleotide repeat loci for this purpose, and for the purpose of making clearer distinctions between MSI-H and MSI-L tumors, as described above (Boland, 1998, supra).
- "MSI-L/S", as used herein, refers to all classified as either MSI-L or MSS.
- "Microsatellite marker", as used herein, refers to a fragment of genomic DNA which includes a microsatellite repeat and nucleic acid sequences flanking the repeat region.
- "Nucleotide", as used herein, refers to a basic unit of a DNA molecule, which includes one unit of a phosphatidyl back bone and one of four bases, adenine ("A"); thymine ("T"); guanine ("G"); and cytosine ("C").
- "Polymerase chain reaction" or "PCR", as used herein, refers to a technique in which cycles of denaturation, annealing with primer, and extension with DNA polymerase are used to amplify the number of copies of a target DNA sequence by approximately 10⁶ times or more. The polymerase chain reaction process for amplifying nucleic acid is covered by U.S. Pat. Nos. 4,683,195 and 4,683,202, which are incorporated herein by reference for a description of the process.
- "Primer", as used herein, refers to a single-stranded oligonucleotide or DNA fragment which hybridizes with a strand of a locus of target DNA in such a manner that the 3' terminus of the primer may act as a site of polymerization using a DNA polymerase enzyme.
- "Primer pair", as used herein, refers to a pair of primers which hybridize to opposite strands a target DNA molecule, to regions of the target DNA which flank a nucleotide sequence to be amplified.
- "Primer site", as used herein, refers to the area of the target DNA to which a primer hybridizes.
- "Stutter", as used herein, refers to a minor fragment observed after amplification of a microsatellite locus, one or more repeat unit lengths smaller than the predominant fragment or allele. It is believed to result from a DNA polymerase slippage event during the amplification process (Levinson & Gutman, 1987 Molecular Biology Evolution 4:203; Schlotterer and Tautz, 1992 Nucleic Acids Research 20:211).

### Design of Primers

Primers for the list of primers according to the present invention were designed for the best combination and are described in the following list:
**SEQ ID NO** 1:TGFBR2F: D3-GAGAAAGAATGACGAGAACATAACAC
**SEQ ID NO** 2:TGFBR2R: TGTTGTCATTGCACTCATCAGA
**SEQ ID NO** 3:BAT26F: D4-TGACTACTTTTGACTTCAGCC
**SEQ ID NO** 4:BAT26R: AACCATTCAACATTTTTAACCC
**SEQ ID NO** S:D17S250F: D4-AAAAGGAAGAATCAAATAGACA
**SEQ ID NO** 6:D17S250R: GCTGGCCATATATATATTTAAACC
**SEQ ID NO** 7:D2S123F: D4-AAACAGGATGCCTGCCTTTA
**SEQ ID NO** 8:D2S123R: GGACTTTCCACCTATGGGAC
**SEQ ID NO** 9:D5S346F: D4-TGGTTTCCATTGTAGCATCTTG
**SEQ ID NO** 10:D5S346R: AATGAAATCGAATGGAGATTGG
**SEQ ID NO** 11:CSF1POF: D4-AACCTGAGTCTGCCAAGGACTAGC
**SEQ ID NO** 12:CSF1POR: TTCCACACACCACTGGCCATCTTC
**SEQ ID NO** 13:BAT25F: D4-TCGCCTCCAAGAATGTAAGT
**SEQ ID NO** 14:BAT25R: TGGCTCTAAAATGCTCTGTTCTC
**SEQ ID NO** 15:MybF: D3-TCCACTTTTCGCCTTTAGGA
**SEQ ID NO** 16:MybR96: CTCCTGCTGCCAGTTTCA
**SEQ ID NO** 17:D18S58F: D3-ATCCCTTAGGAGGCAGGAAA
**SEQ ID NO** 18:D18S58R: CTCCCGGCTGGTTTTATTTA
**SEQ ID NO** 19:MT1XF: D4-GACAGCTGTGCTCTCAGATGTAAA
**SEQ ID NO** 20:MT1XR: CCAAGTGCCATATACCCAGTGA
**SEQ ID NO** 21:BAT40F: D4-GCTTCCAGTCCTCATCGAAACT
**SEQ ID NO** 22:BAT40R: GTAGAGCAAGACCACCTTG
**SEQ ID NO** 23:D7S820F: D4-TGTCATAGTTTAGAACGAACTAAC
**SEQ ID NO** 24:D7S820R: TGAGGTATCAAAAACTCAGAGG
**SEQ ID NO** 25:D18S51F: CAAACCCGACTACCAGCAAC
**SEQ ID NO** 26:D18S51R: GAGCCATGTTCATGCCACTG

The forward primer of each microsatellite locus should be labeled by a fluorophore able to be detected by the capillary electrophoresis use such as Applied Biosystems 310, 3100, 3130 or similar, or Beckman Coulter CEQ2000XL, CEQ8000, CEQ8800. Fluorophores are suggested in Table 1. For use with SpreadEx ® el (Elchrom) and intercalator dyes such as SYBRGreen, GelStar or analogs, the fluorescent labelling is not necessary.

### Design and Testing of MSI Multiplex

The method of multiplex analysis of microsatellite loci of the present invention contemplates selecting an appropriate set of loci, primers, and amplification protocols to generate amplified alleles from multiple co-amplified loci which preferably do not overlap in size or, more preferably, which are labeled in a way which enables one to differentiate between the alleles from different loci which overlap in size. Combinations of loci may be rejected for either of the above two reasons, or because, in combination, one or more of the loci do not produce adequate product yield, or fragments which do not represent authentic alleles are produced in this reaction. The following factors are preferably taken into consideration in deciding upon which loci to include in a multiplex of the present invention. To effectively design the microsatellite multiplex, size ranges for alleles at each locus are determined. This information is used to facilitate separation of alleles between all the different loci, since any overlap could result in an allele from one locus being inappropriately identified as instability at another locus.

It is also contemplated that other factors, such as successful combinations of materials and methods, are taken into consideration in designing a multiplex of MSI loci. Determination of such additional factors can be determined by following the selection methods and guidelines disclosed herein, and by using techniques known to one of ordinary skill in the art of the present invention. Specifically, the same or substantially similar techniques can be used to identify the preferred MSI loci and sets of MSI loci described herein below to select primer pair sequences, and to adjust primer concentrations to identify an equilibrium in which all included loci may be amplified. In other words, once the method and materials of this invention are disclosed, various methods of selecting loci, primer pairs, and amplification techniques for use in the method and kit of this invention are likely to be suggested to one skilled in the art. All such methods are intended to be within the scope of the present claims.

### F. Sources of Genomic DNA

The genomic DNA amplified or co-amplified according to the methods of the present invention originates from biological material from a human individual subject. The biological material can be any tissue, cells, or biological fluid from the subject which contains genomic DNA. The biological material is preferably selected from the group consisting of tumor tissue, disseminated cells, feces, blood cells, blood plasma, serum, lymph nodes, urine, and other bodily fluids.

The biological material can be in the form of tissue samples fixed in formalin and embedded in paraffin (FFPE). Tissue samples from biopsies are commonly stored in FFPE for long term preservation. Formalin creates cross-linkages within the tissue sample which can be difficult to break, sometimes resulting in low DNA yields. Another problem associated with formalin-fixed paraffin-embedded samples is amplification of longer fragments is often problematic. When DNA from such samples is used in multiplex amplification reactions, a significant decrease in peak heights is seen with increasing fragment size. The microsatellite analysis method and kit of the present invention are preferably designed to amplify and analyze DNA from FFPE tissue samples.

### Design of the assay

This assay needs normal and tumor DNA. Amplicons from 2 multiplex PCR for normal DNA and 2 multiplex PCR for tumor DNA (total of 4 multiplex PCR) will be loaded to a capillary electrophoretic apparatus in order to compare the exact length of 13 microsatellite markers obtained from normal tissue and tumor tissue of the same case. Alternatively the PCR products should be loaded to the high resolution gel Spreadex® from Elchrom (Swisse).

### Internal Controls:

Three tetranucleotide markers are use to check for possible contamination.

CSF1PO, D18S51 and D7S820 are highly polymorphic tetranucleotide markers usually stable even for MSI-H cases. Normal and tumor DNA from the same patient should have the same alleles. In a few cases these markers might display loss of heterozygosity for these three loci but at least one allele should be the same.

**Cross-contamination:** during evaluation tests, 20 runs were tested alternating MSI-H and MSS samples without having false positive results

### Specificity of the assay

In 1997, a National Cancer Institute (NCI) workshop recommended a panel of five microsatellite markers for detection of MSI consisting of two mononucleotide markers and three dinucleotide repeats. Samples with instability in two or more of these markers are defined as MSI-High (MSI-H), whereas those with one unstable marker are designated as MSI-Low (MSI-L). The use of ten microsatellite markers allows to reach the best specificity as indicated by Umar et Al.( Umar A, et Al. Revised Bethesda Guidelines for hereditary nonpolyposis colorectal cancer (Lynch syndrome) and microsatellite instability. J Natl Cancer Inst. 2004 Feb 18;96(4):261-8.)

### Sensitivity

The analytical detection limit (sensitivity limit) of the AlphaCapillary MSI *Kit* was determined on the Beckman Coulter CEQ 2000™ Instrument. The detection limit is 5% microsatellite unstable cells in a background of 95% of normal stable ones.

## Claims

1. A method of analyzing micro-satellite loci, comprising steps of: a) providing primers for co-amplifying a set of at least 13 microsatellite loci of human genomic DNA, comprising at least six mononucleotide repeat loci selected from the group consisting of BAT-25, BAT-26, BAT-40, MybT22, TGF-Beta R2 and MT1XT20 and at least four dinucleotide repeat loci selected from the group consisting of D18S58, D2S123, D5S346, D17S250 and at least three tetranucleotide repeat loci selected from the group consisting of D18S51, D7S820 and CSF1PO; b) co-amplifying the set of at least six microsatellite loci from at least one sample of genomic DNA in a multiplex amplification reaction, using at least one oligonucleotide primer for each locus which is fluorescently labeled, thereby producing amplified DNA fragments; and c) determining the size of the amplified DNA fragments.

2. The method of claim 1, wherein the at least one sample of genomic DNA comprises a first sample of genomic DNA originating from normal non-cancerous biological material from an individual and a second sample of genomic DNA originating from a tumor of the individual the method further comprising: detecting microsatellite by comparing the size of the amplified DNA fragments produced from co-amplifying the first sample of genomic DNA to the size of the amplified DNA fragments produced from co-amplifying the second sample of genomic DNA.

3. The method of claim 2, wherein the microsatellite instability results are used in prognostic tumor diagnosis.

4. The method of claim 2, wherein the microsatellite instability (MSI) results are used in the diagnosis of familial tumor predisposition.

5. The method of claim 2, wherein the microsatellite instability results are used to detect cancerous tumors of the gastro-intestinal system and of the endometrium.

6. The method of claim 5, wherein the cancerous tumors are tumors from a colorectal cancer.

7. The method of claim 1, wherein at least one of the primers provided in step (a) has a nucleic acid sequence selected from the group of primer sequences identified by: SEQ ID NO: 1-26.

8. The method for assessment of the MSI status of medically relevant conditions associated with MSI comprising determining the presence or absence of mutations in the T-20 repeat located in the 3'-UTR of the MI1X gene and assessing the MSI status from said presence or absence of mutations in the T-20 located in the 3'-UTR of the MT1X gene, wherein the presence of mutations is indicative for the presence of a MSI-H phenotype.

9. The method of Claim 8, wherein the medically relevant condition is a tumor.

10. The method of Claim 8, wherein the tumor is selected from a group comprising tumors of the anogenital tract, tumors of the colorectal tract, colorectal carcinoma, colorectal adenoma, colorectal polyps, tumors of the gastrointestinal tract, carcinoma of the small intestine, polyps of the small intestine, tumors of the bile ducts, pancreatic carcinoma, tumors of the respiratory tract, endometrial tumors, endometrial carcinoma, endometrial hyperplasia, tumors of the central nervous system, Glioblastoma multiforme, medulloblastoma, tumors of the prostate, tumors of the breast carcinoma.

11. The method of any one of Claims 8 to 10, wherein the determining of the presence or absence of mutations in the T-20 repeat located in the 3'-UTR of the MT1X gene is carried out using nucleic acids probes or primers as binding agents for the detection reaction,

12. The method of any one of the preceding Claims, wherein the detection reaction is carried out using a nucleic acid hybridization reaction or a nucleic acid amplification reaction.

13. A kit for performing a method for assessment of the MSI status of medically relevant conditions associated with MSI comprising one or more binding agents specifically recognizing a MT1X nucleic acid.

14. The kit of Claim 13, wherein at least one binding agent is a nucleic acid probe or primer.

15. The kit according to Claim 13 or 14 wherein the probes or primers are selected from SEQ ID NO: 1-26.
